Europäisches Patentamt

European Patent Office

Office européen des brevets

(11) Publication number: **0 278 626**
**A1**

# (12) EUROPEAN PATENT APPLICATION

(21) Application number: **88300632.2**

(22) Date of filing: **26.01.88**

(51) Int. Cl.⁴: **A61G 3/00**

(30) Priority: **31.01.87 JP 21462/87**

(43) Date of publication of application:
**17.08.88 Bulletin 88/33**

(84) Designated Contracting States:
**DE FR GB IT SE**

(71) Applicant: **Ishii, Yasuhiko**
**3-42 Ikego 2-chome**
**Zushi-shi Kanagawa-ken 249(JP)**

(72) Inventor: **Ishii, Yasuhiko**
**3-42 Ikego 2-chome**
**Zushi-shi Kanagawa-ken 249(JP)**

(74) Representative: **Calderbank, Thomas Roger et al**
**MEWBURN ELLIS & CO. 2/3 Cursitor Street**
**London EC4A 1BQ(GB)**

(54) **Mobile odontological vehicle.**

(57) A mobile odontological vehicle of this invention has an engine and a steering function which enable an operator to go to the house of a patient who cannot go to a dental clinic. The patient who is sitting in a wheelchair can be lifted into the vehicle (1) by utilizing a power gate (25) driven by a generator (12) mounted in the vehicle. The patient is then examined by using an odontological unit (7), a medicine cabinet (14) and an X-ray unit (9) and so on, and is treated by using the odontological unit, the medicine cabinet, a dental instrument cabinet (13), a compressor (24) and so on.

**F I G. 2(a)**

EP 0 278 626 A1

## MOBILE ODONTOLOGICAL VEHICLE

The present invention relates to a mobile treatment vehicle, and, more particularly, to a mobile odontological vehicle loaded with dental instruments and a generator.

Mobile vehicles designed to provide first aid or medical examinations, such as ambulances, X-ray vehicles, blood collection vehicles, and cancer examination vehicles, are in practical use. However, these vehicles are not aimed at treating illness.

An ambulance which rapidly carries a patient to a nearby hospital is usually loaded only with a bed on which the patient is laid and medical devices which are necessary for giving first aid to the patient, such as stanching bleeding. X-ray vehicles, blood collection vehicles, and cancer examination vehicles are each loaded only with necessary medical instruments and a bed.

As the trend toward nuclear families has proceeded and the population of elderly people has increased, the number of patients who cannot go to see a doctor has also increased.

For patients suffering from internal diseases, the system by which a doctor makes calls on the patients on their own homes is well known. However, there is no system for providing the dentistry which requires bulky, heavy instruments.

Recently, there have been demands for dentists to pay calls on elderly patients who happen to be bedridden. There are also demands for treatment by dentists at locations where no commercial power source is available.

Accordingly, a primary object of the present invention is to provide a mobile odontological vehicle which enables a dentist or a dental technician to go and see elderly bedridden patients at their own homes and give dental treatment to them.

A second object of the present invention is to provide a mobile odontological vehicle which enables dental treatment to be performed at locations where no commercial power source is available.

A third object of the present invention is to provide a mobile odontological vehicle which is provided with means for allowing an elderly bedridden patient to be lifted into the vehicle together with a wheelchair so as to enable him to receive dental treatment without requiring another person's help.

The first object is achieved by the provision of a vehicle which is a self-propelled vehicle divided into an operator's chamber and a treatment chamber by a partition wall; the treatment chamber containing an odontological unit, a medicine cabinet, a dental instrument cabinet, a power source cell, an X-ray unit, and a ventilating fan; the partition wall and side walls of the treatment chamber being provided with plates that do not transmit X-rays; the operator's chamber containing a controller for the X-ray unit; and a compressor and a water-supply/drainage tank being provided under a floor plate.

The second object is achieved by the provision of a generator in the vehicle employed to achieve the first object.

The third object is attained by the provision of a power gate on a rear door side of the self-propelled vehicle. In the drawings:

Figs. 1 (a) and (b) are cross-sectional views of the essential parts of a mobile odontological vehicle;

Figs. 2 (a) and (b) are side elevational views of the the vehicle, with the essential parts thereof being shown in section

Fig. 3 is a perspective view of an odontological unit; and

Figs. 4 (a), (b), 5, 6 (a), (b), 7 and 8 illustrate how a patient is lifted in by using a power gate.

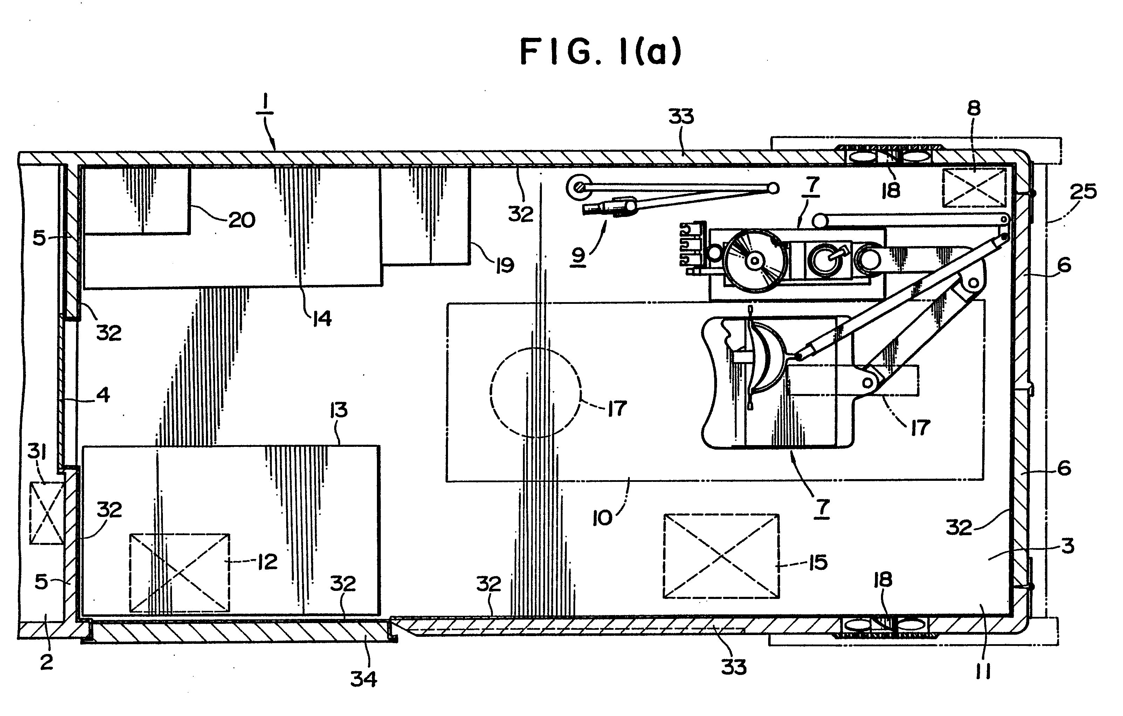

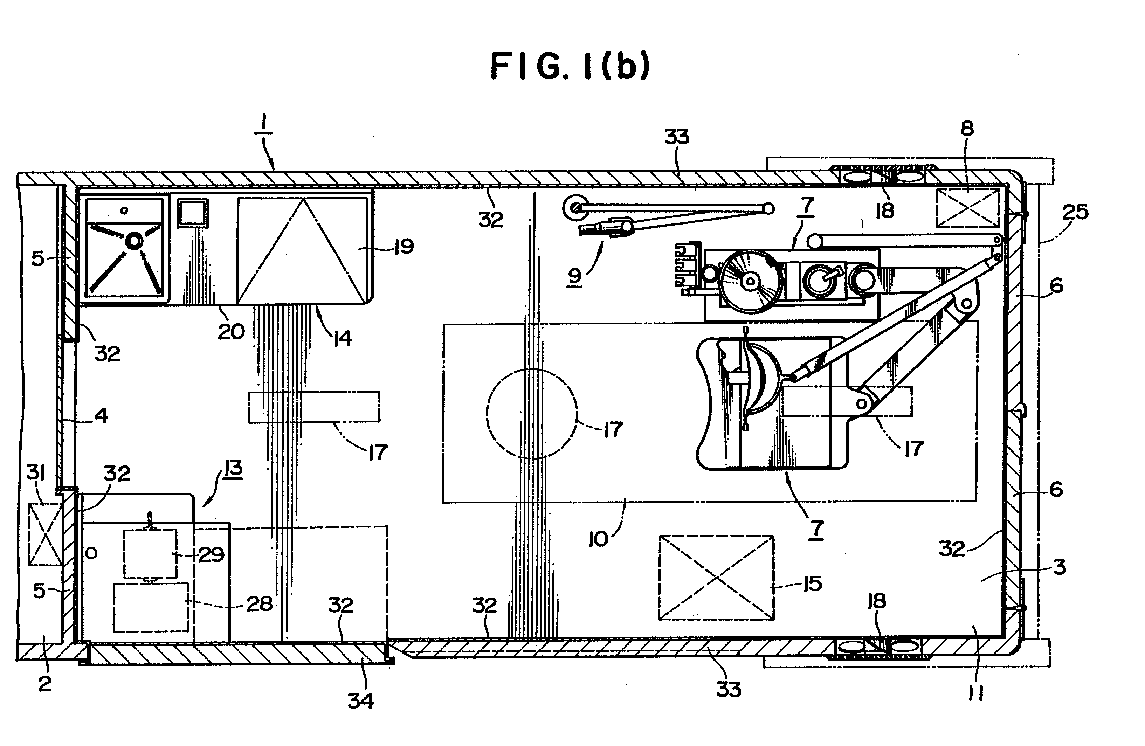

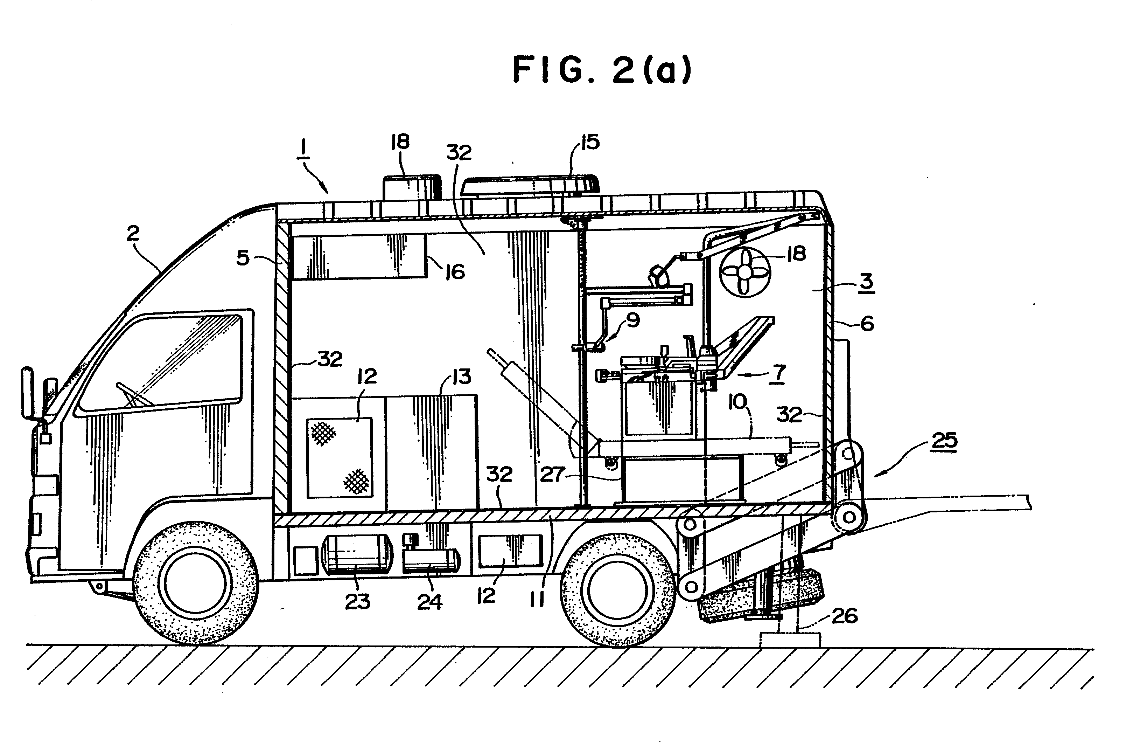

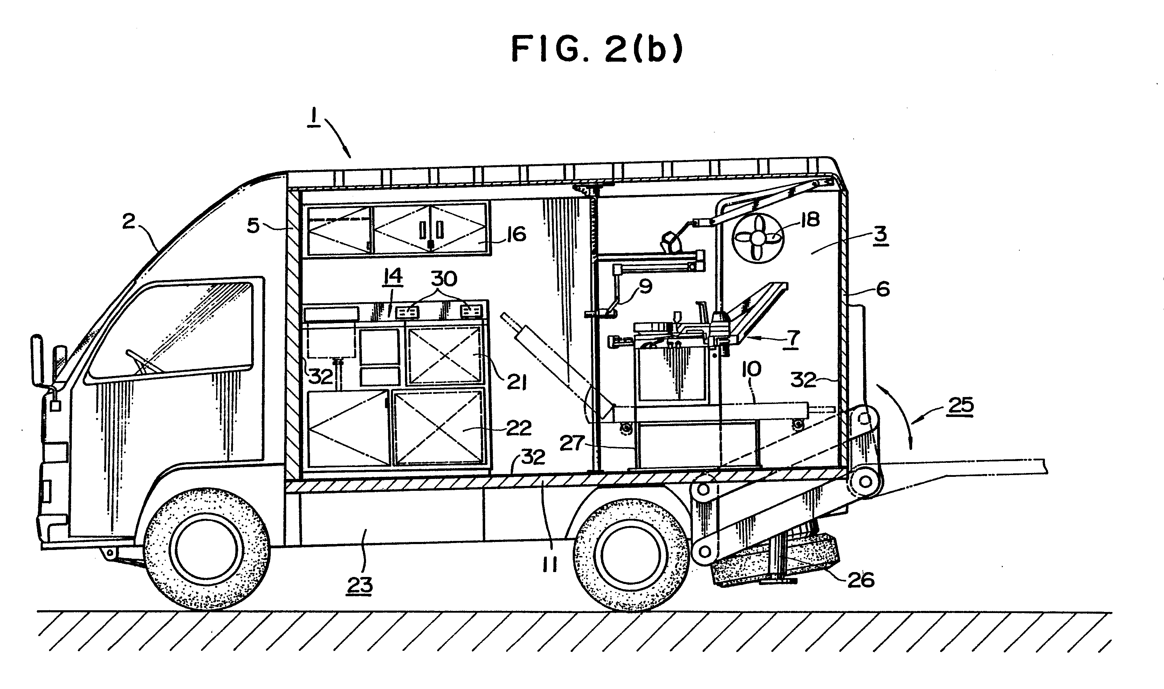

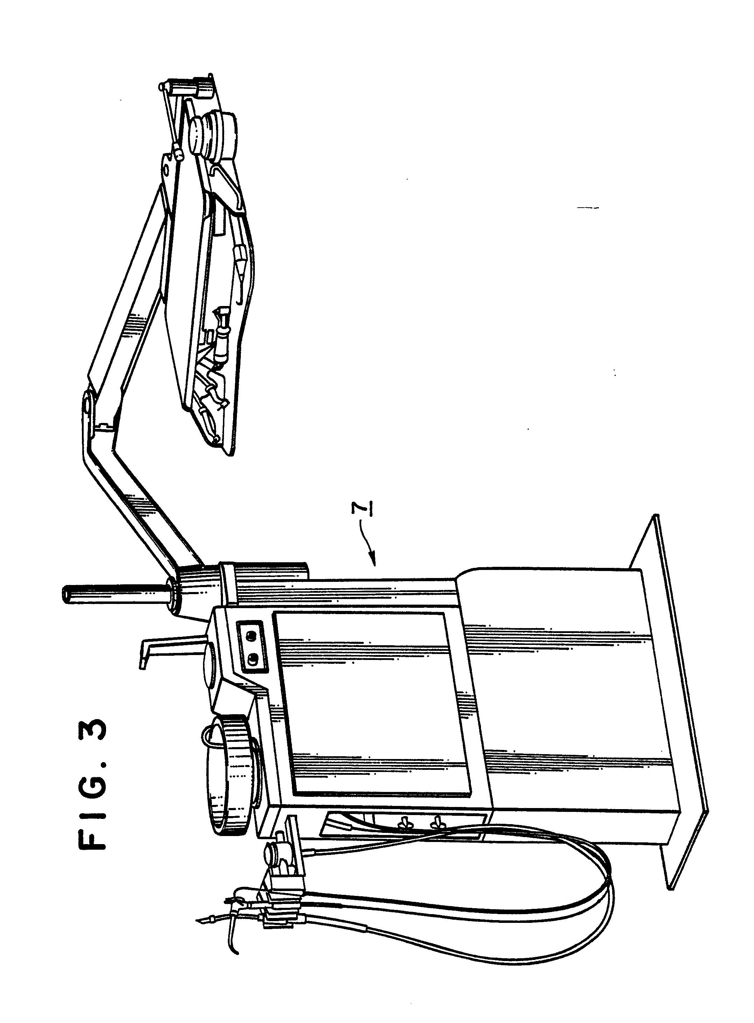

A mobile odontological vehicle of this embodiment is a two-box type of self-propelled vehicle 1 which is divided into an operator's chamber 2 and a treatment chamber 3 by a partition wall 5 provided with a door 4, as shown in Figs. 1 (a) and 2 (a). An odontological unit 7, such as that shown in Fig. 3, a vacuum unit 8 for treatment, an X-ray unit 9 and a treatment bed 10 are provided on a floor 11 of the treatment chamber 3 near a rear door 6, with the weight balance of these units being taken into consideration.

On the floor plate 11 of the treatment chamber 3 closer to the partition wall 5 that separates the operator's chamber 2 from the treatment chamber are disposed a generator 12 which can be connected to a commercial power source, but which is separate from the already provided DC generator of the vehicle, a dental instrument cabinet 13, and a medicine cabinet 14.

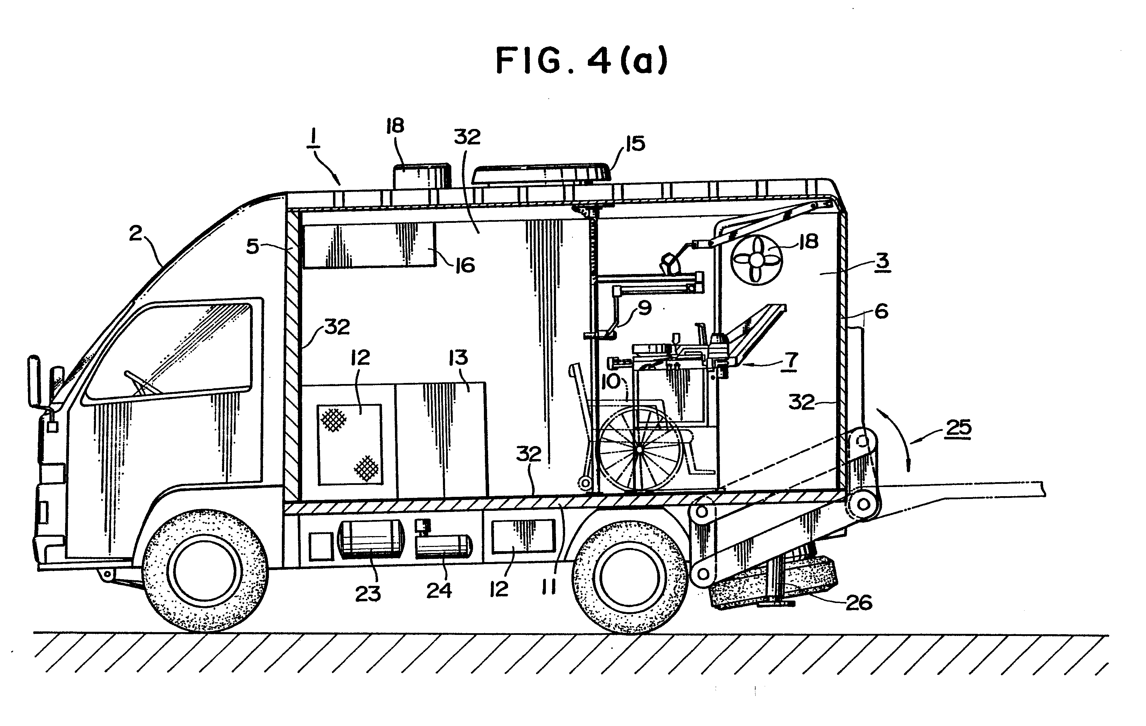

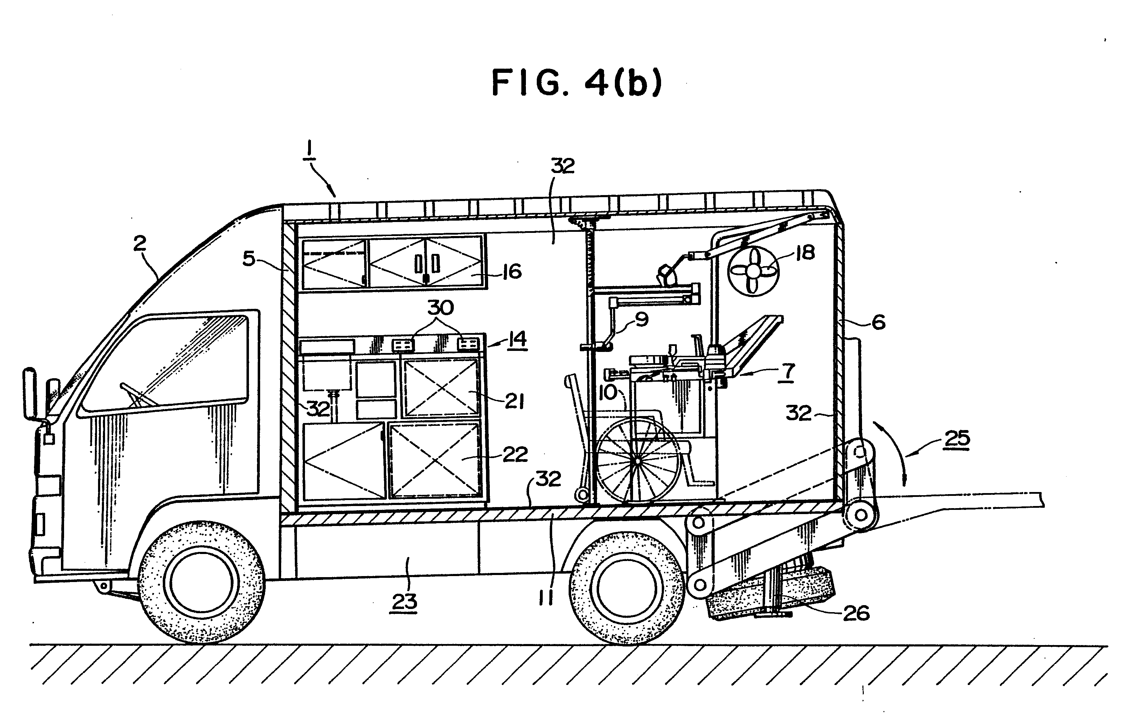

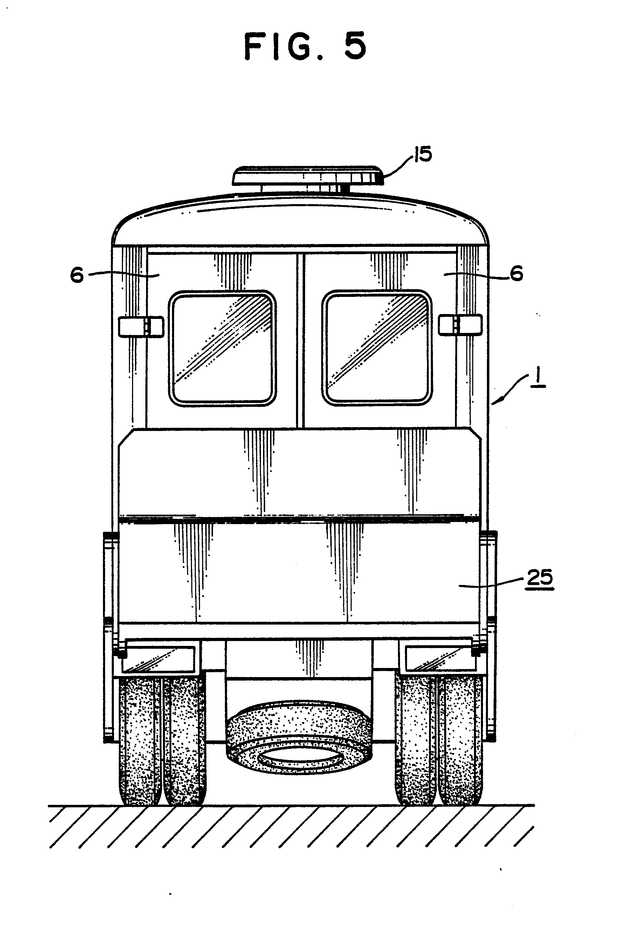

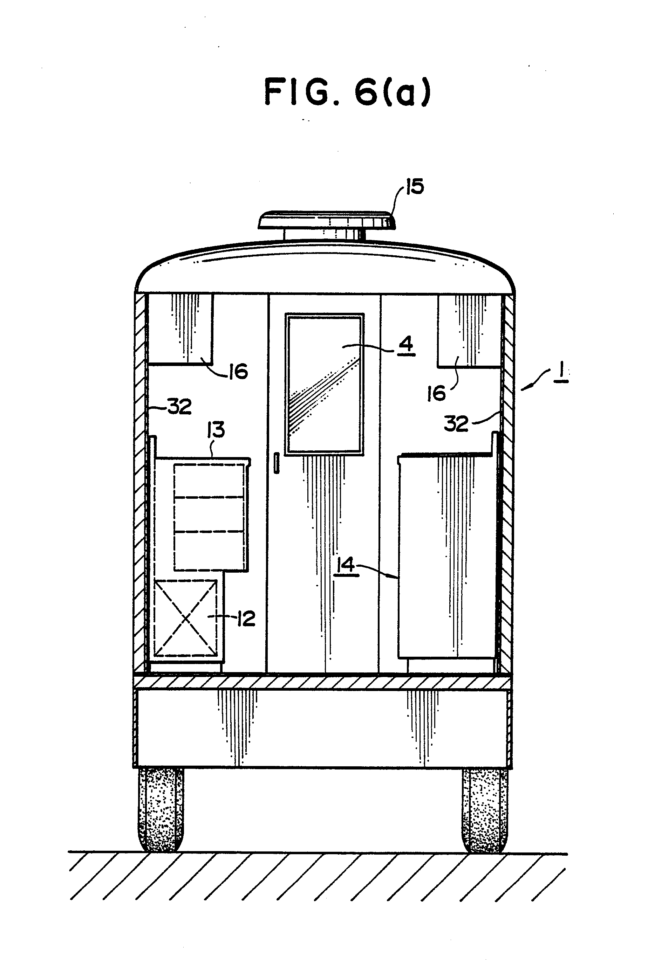

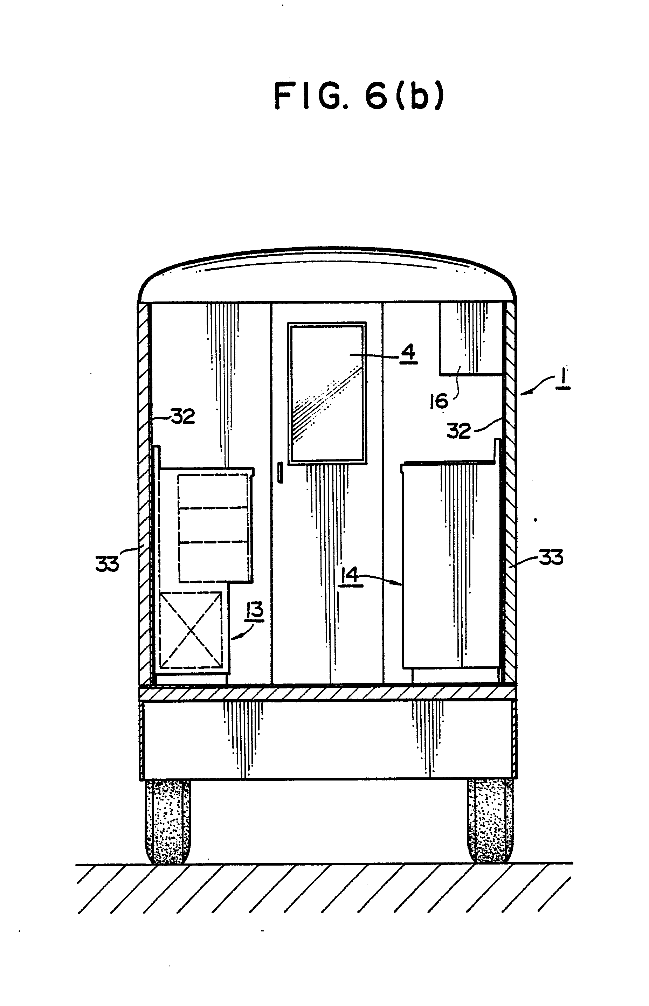

As shown in Figs. 4 (a) to 6 (a), an air conditioner 15, hanging racks 16 for supporting medicines, fluorescent lamps 17, and a ventilating fan 18 are provided on the ceiling. A preparation cabinet 19 and a rack 20 with a storage case, an ultrasonic washer, and a dry sterilizer are further provided on the floor plate 11, and a water-supply/drainage tank 23 and a compressor 24 are disposed under the floor.

Outside the rear door 6 are provided a power gate 25 and an outrigger 26 (a leg for fixing the car body) by known means.

A controller 31 for the X-ray unit 9 is disposed within the operator's chamber 2.

The ceiling, floor, and walls of the treatment chamber 3, the partition wall 5 and the doors 4 and 6 are provided with plates 26 that do not transmit the X-rays, such as a poly-plywood containing lead, so as to prevent any leakage of the X-rays.

The generator 12 may be disposed under the floor, and the ventilating fan 18 may be disposed on a wall, as shown in Figs. 1 (a) and 2 (a).

If the DC generator which is already provided in the self-propelled vehicle 1 is a relatively large one, the generator 12 may be replaced by an DC/AC converter.

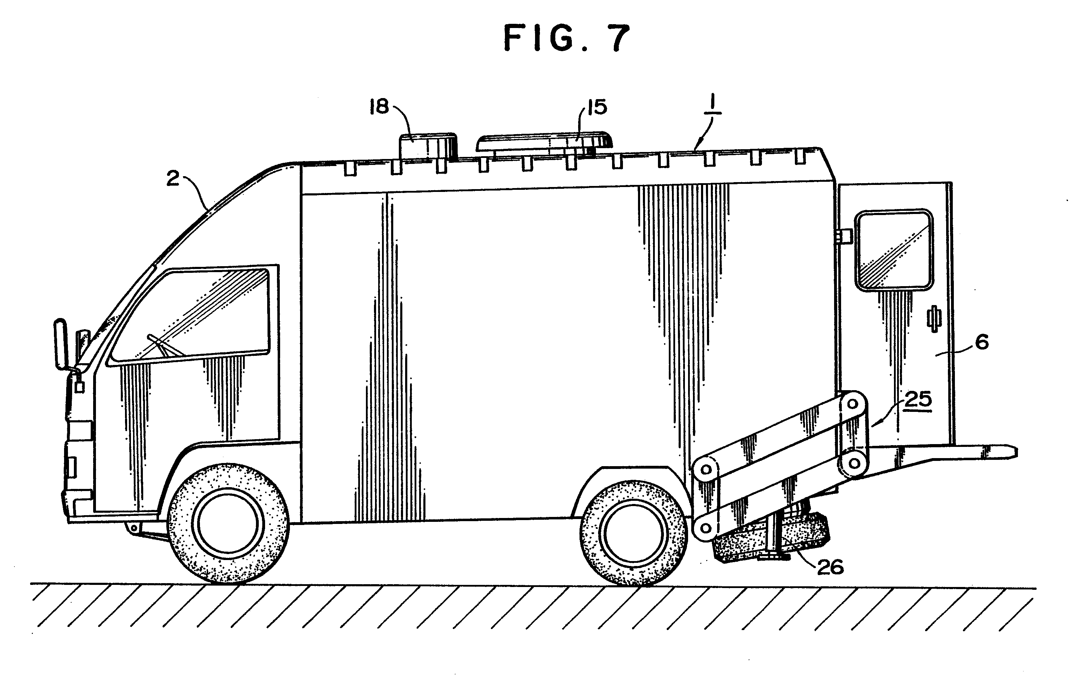

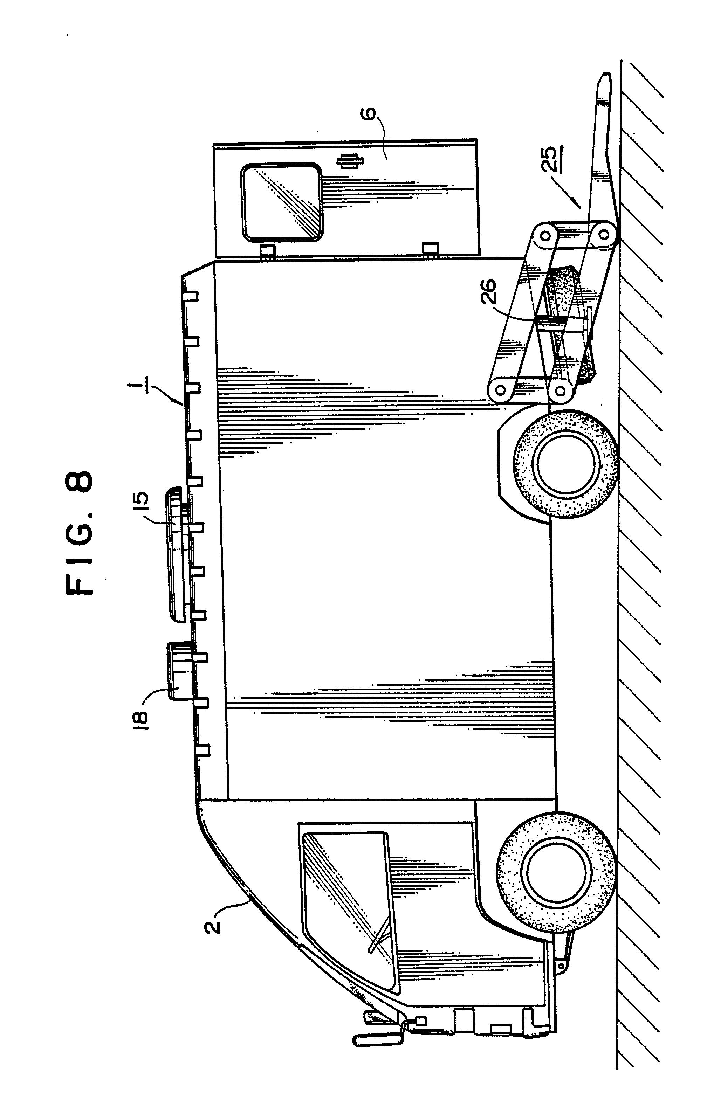

When requested by a patient who cannot visit the dental clinic, the operator drives the thus-arranged mobile odontological vehicle to the patient's house and positions the car body on the extended outrigger 26. If the patient has difficulty walking, the operator lowers the power gate 25 to the ground, as shown in Figs. 7 and 8, and lifts the patient who is sitting in a wheelchair or a wheelchair-type treatment bed 10 into the treatment chamber 3. The patient may alternatively be laid on a stretcher-type treatment bed 10, the bed 10 being carried in on a base 27, as shown by broken lines in Fig. 2 (b).

Thereafter, the rear door 6 is closed and the patient is treated with the various dental instruments.

If radiography is needed, the operator sets the X-ray unit 9 at the appropriate part of the patient, and operates the controller 31 of the X-ray unit in the operator's chamber to take a radiograph, the controller being provided in the operator's chamber so as to ensure that the opertor is not exposed to X-rays.

Once the treatment is completed, the rear door 6 is opened, and the patient is lifted out by utilizing the power gate 25. After the outrigger 26 is collapsed, the vehicle is ready to move on to the next patient.

As will be understood from the foregoing description, since the mobile odontological vehicle of the present invention adopts means for solving the above-described problems, it enables a dentist or a dental technician to visit patients' homes to give dental treatment using various heavy dental instruments. The generator mounted in the vehicle enables dental treatment to be performed at locations where no commercial power source is available. The poly-plywood that contains lead and is attached to the walls prevents any leakage of X-rays. If a radio is mounted in the vehicle, the dentist or the dental techinician is able to go to a next patient's house directly from the house of the previous patient so as to relieve the pain of the next patient in the quickest time. In particular, an elderly bedridden patient can be easily lifted into the vehicle by using the power gate, thus facilitating dental treatment of old people.

Another embodiment of the present invention will be described below. A mobile odontological vehicle of this embodiment is a two-box type of self-propelled vehicle 1 which is divided into an operator's chamber 2 and a treatment chamber 3 by a partition wall 5 provided with a door 4. Inside the treatment chamber 3, an odontological unit 7, such as that shown in Fig. 3, a vacuum unit 8 for treatment, an air conditioner 15 for the treatment chamber, an X-ray unit 9 and a treatment bed 10 are provided on a floor 11 of the treatment chamber 3 closer to a rear door 6, with the weight balance of these units being taken into consideration. On a step and the floor plate 11 of the treatment chamber 3 near a side door 34 and the partition wall 5 that separates the operator's chamber 2 from the treatment chamber are disposed a compressor 24, a dental instrument cabinet 13, a handy dust collector 28, a dental lathe 29 (a uniroll) and a medicine cabinet 14. A hanging rack 16 for supporting medicines is provided above the cabinet 14. A preparation cabinet 19 and a rack 20 with a storage case, an ultrasonic washer 21 and a dry sterilizer 22 are provided on the floor plate 11 of the treatment chamber opposite from the side door side. A water-supply/drainage tank 23 is disposed under the floor, and fluorescent lamps 17 and a ventilating fan 18 are mounted on the ceiling. A power gate 25 is provided outside the rear door 6, and a controller 31 for the X-ray unit 9 is disposed within the operator's chamber 2. The ceiling, floor and walls of the treatment chamber 3, the partition wall 5 and the door 4 are provided with plates 32 that do not transmit X-rays, such as a poly-plywood containing lead, so as to prevent any leakage of the X-rays. The treatment chamber is constructed by a side wall 33 and the floor plate 11. Reference numerals 30 in Fig. 2 denote power outlets.

When requested by a patient who cannot visit the dental clinic, the operator drives the thus-arranged mobile odontological vehicle to the patient's house. If the patient has difficulty walking, the operator lowers the power gate 25 to the ground, as shown in Figs. 4 (b) to 8, and lifts the patient who is sitting in a wheelchair or a wheelchair-type treatment bed 10 into the treatment chamber 3. The patient may alternatively be laid on a stretcher-type treatment bed 10, the bed 10 being carried in on a base 27, as shown by broken lines in Fig. 2 (b).

Thereafter, the rear door 6 is closed and the patient is treated with the various dental instruments. If radiography is needed, the operator sets the X-ray unit 9 at the appropriate part of the patient, and operates the controller 31 of the X-ray unit in the operator's chamber to take a radiopraph, the controller being provided in the operator's chamber so as to ensure that the operator is not exposed to X-rays.

Once the treatment is completed, the rear door 6 is opened, and the patient is lifted out by utilizing the power gate 25 so as to get the vehicle ready for the next patient.

## Claims

1. A mobile odontological vehicle comprising a self-propelled vehicle divided into an operator's chamber and a treatment chamber by a partition wall, said treatment chamber containing an odontological unit, a medicine cabinet, a dental instrument cabinet, a power source cell, an X-ray unit and a ventilating fan, said partition wall and side walls of said treatment chamber being provided with plates that do not transmit X-rays, said operator's chamber containing a controller for said X-ray unit, and a compressor and a water-supply/drainage tank being provided under a floor plate.

2. A mobile odontological vehicle according to claim 1, wherein said self-propelled vehicle contains a generator so as to enable power needed for treatment to be obtained therefrom.

3. A mobile odontological vehicle according to claim 2, wherein said generator is provided under the floor of said self-propelled vehicle.

4. A mobile odontological vehicle according to claims 1 and 2, wherein the rear door side of said self-propelled vehicle is provided with a power gate which enables a patient to be lifted into said vehicle.

5. A mobile odontological vehicle according to claim 1, wherein said self-propelled vehicle is provided with a DC/AC converter for converting the DC power of said power source cell into AC power so as to enable power required for treatment to be obtained therefrom.

# FIG. I(a)

0 278 626

FIG. I(b)

# FIG. 2(a)

0 278 626

# FIG. 2(b)

0 278 626

# FIG. 3

7

0 278 626

# FIG. 4(a)

# FIG. 4(b)

0 278 626

# FIG. 5

# FIG. 6(a)

# FIG. 6(b)

# FIG. 7

0 278 626

# FIG. 8

0 278 626

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 090 300 (RETHMEIER) <br> * claim 1; figure 1 * <br> --- | 1,2 | A 61 G 3/00 |
| A | EP-A-0 089 932 (POY) <br> * claims 1, 2; figure 1 * <br> --- | 1,2 | |
| A | BE-A- 420 412 (JONCKHEERE & FLORIZOONE, STE N.C.) <br> * claims 1-4; page 2, lines 14-18 * <br> --- | 1-3,5 | |
| A | EP-A-0 048 206 (BOURGEOIS) <br> * claim 1 * <br> ----- | 1,4 | |
| | | | TECHNICAL FIELDS SEARCHED (Int. Cl.4) |
| | | | A 61 B 6/00 <br> A 61 G 3/00 <br> B 60 P 3/00 |

The present search report has been drawn up for all claims

| Place of search | Date of completion of the search | Examiner |
|---|---|---|
| BERLIN | 15-04-1988 | PAPA E.R. |